(19) Europäisches Patentamt
European Patent Office
Office européen des brevets

(11) EP 0 722 711 B1

(12) FASCICULE DE BREVET EUROPEEN

(45) Date de publication et mention de la délivrance du brevet: **19.02.1997 Bulletin 1997/08**

(51) Int. Cl.$^6$: **A61K 7/13**

(21) Numéro de dépôt: **96400045.9**

(22) Date de dépôt: **08.01.1996**

# (54) Composition de teinture d'oxydation des fibres kératiniques et procédé de teinture mettant en oeuvre cette composition

Keratinische Fasern, Oxidationsfärbemittel sowie Färbungsverfahren unter Verwendung dieser Zusammensetzung

Keratinous fiber oxidation dyeing composition and the dyeing process using this composition

(84) Etats contractants désignés:
**AT BE CH DE DK ES FR GB GR IE IT LI LU MC NL PT SE**

(30) Priorité: **20.01.1995 FR 9500663**

(43) Date de publication de la demande: **24.07.1996 Bulletin 1996/30**

(73) Titulaire: **L'OREAL**
**75008 Paris (FR)**

(72) Inventeurs:
- **Audousset, Marie-Pascale**
**F-92600 Asnières (FR)**
- **Cotteret, Jean**
**F-78480 Verneuil sur Seine (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 428 441** **EP-A- 0 465 340**

EP 0 722 711 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.13.14/3.4

## Description

La présente invention a pour objet une composition pour la teinture d'oxydation des fibres kératiniques, en particulier des fibres kératiniques humaines telles que les cheveux, comprenant un précurseur de colorant d'oxydation convenablement sélectionné, en association avec le 4-hydroxyindole, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques comme le 4-hydroxyindole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet allemand DE 3 031 709, des compositions pour la teinture d'oxydation à pH basique des fibres kératiniques, renfermant une base d'oxydation telle que la paraphénylènediamine ou le para-aminophénol, et du 4-hydroxyindole à titre de coupleur. De telles compositions ne sont cependant pas entièrement satisfaisantes, notamment au niveau de la tenue des colorations obtenues vis à vis des agents extérieurs susmentionnés.

Il a également déjà été proposé, notamment dans le brevet français FR 2 664 304, des compositions pour la teinture d'oxydation à pH acide des fibres kératiniques, renfermant au moins un précurseur de colorant d'oxydation tel que le para-aminophénol ou la paraphénylènediamine et du 4-hydroxyindole à titre de coupleur. De telles compositions ne sont pas non plus entièrement satisfaisantes, notamment au niveau de la sélectivité des colorations obtenues.

Or, la demanderesse vient maintenant de découvrir qu'il est possible d'obtenir de nouvelles teintures particulièrement résistantes, notamment à la transpiration, qui engendrent des colorations intenses et peu sélectives, en associant au moins un précurseur de colorant d'oxydation choisi parmi le 3-fluoro 4-aminophénol et le N,N'-bis-(β-hydroxyéthyl) N,N'-bis-(4'-aminophényl) 1,3-diamino 2-propanol et leurs sels d'addition avec un acide, avec du 4-hydroxyindole à titre de coupleur.

Cette découverte est à la base de la présente invention.

L'invention a donc pour objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins un précurseur de colorant d'oxydation choisi parmi le 3-fluoro 4-aminophénol, le N,N'-bis-(β-hydroxyéthyl) N,N'-bis-(4'-aminophényl) 1,3-diamino 2-propanol et leurs sels d'addition avec un acide ;
- du 4-hydroxyindole, à titre de coupleur.

Les colorations obtenues avec la composition conforme à l'invention présentent une bonne puissance tinctoriale et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition.

Les sels d'addition avec un acide utilisables dans le cadre des compositions tinctoriales de l'invention sont notamment choisis parmi les chlorhydrates, les bromhydrates, les sulfates et les tartrates.

L'ensemble des précurseurs de colorants d'oxydation conformes à l'invention, c'est à dire le 3-fluoro 4-aminophénol et/ou le N,N'-bis-(β-hydroxyéthyl) N,N'-bis-(4'-aminophényl) 1,3-diamino 2-propanol et/ou le ou leurs sels d'addition avec un acide, représente de préférence de 0,0005 à 10 % en poids environ du poids total de la composition tinctoriale, et encore plus préférentiellement de 0,01 à 5 % en poids environ.

Le 4-hydroxyindole représente de préférence de 0,0001 à 3,5 % en poids environ du poids total de la composition

tinctoriale, et encore plus préférentiellement de 0,005 à 1 % en poids environ.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale telle que définie précédemment est généralement compris entre 3 et 12. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (I) suivante :

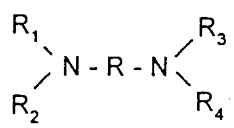

(I)

dans laquelle R est un reste propylène éventuellement substitué par un groupement hydroxyle ou un radical alkyle en $C_1$-$C_4$ ; $R_1$, $R_2$, $R_3$ et $R_4$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 40 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents

acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**EXEMPLES 1 ET 2**

On a préparé les compositions tinctoriales 1 et 2 conformes à l'invention, suivantes (teneurs en grammes) :

| COMPOSITION | 1 | 2 |
|---|---|---|
| 3-fluoro 4-aminophénol | 0,528 | |
| Tétrachlorhydrate de N,N'-bis-(β-hydroxyéthyl) N,N'-bis-(4'-aminophényl) 1,3-diamino 2-propanol | | 0,4 |
| 4-hydroxyindole | 0,520 | 0,4 |
| Support de teinture commun (*) | (*) | (*) |
| Eau q.s.p. | 100 g | 100 g |

(*) support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |

| | | |
|---|---|---|
| - Alcool éthylique | 7,0 | g |
| - Dipropylèneglycol | 0,5 | g |
| - Monométhyléther de propylèneglycol | 9,0 | g |
| - Métabisulfite de sodium en solution aqueuse, à 35 % de M.A. | 0,455 | g M.A. |
| - Acétate d'ammonium | 0,8 | g |
| - Antioxydant, séquestrant | q.s. | |
| - Parfum, conservateur | q.s. | |
| - Ammoniaque à 20 % de $NH_3$ | 10,0 | g |

Au moment de l'emploi, on a mélangé chaque composition tinctoriale avec une quantité égale d'une composition oxydante constituée par une solution d'eau oxygénée à 20 volumes (6 % en poids).

Chaque mélange résultant a été appliqué pendant 30 minutes sur des mèches de cheveux gris à 90 % de blancs, naturels ou permanentés. Les mèches de cheveux ont ensuite été rincées, lavées avec un shampooing standard puis séchées.

Les mèches de cheveux ont été teintes dans les nuances figurant dans le tableau ci-dessous :

| EXEMPLE (COMPOSITION) | NUANCE SUR CHEVEUX NATURELS | NUANCE SUR CHEVEUX PERMANENTES |
|---|---|---|
| **1 (1)** | irisé cuivré léger | irisé cuivré |
| **2 (2)** | cendré bleuté léger | bleu cendré |

**EXEMPLE 3**

On a préparé la composition tinctoriale conforme à l'invention, suivante :

| | |
|---|---|
| - Tétrachlorhydrate de N,N'-bis-(β-hydroxyéthyl) N,N'-bis-(4'-aminophényl) 1,3-diamino 2-propanol | 1 g |
| - 4-hydroxyindole | 0,26 g |
| - Alcool oléique polyglycérolé à 2 moles de glycérol | 4,0 g |
| - Alcool oléique polyglycérolé à 4 moles de glycérol, à 78 % de matières actives (M.A.) | 5,69 g M.A. |
| - Acide oléique | 3,0 g |
| - Amine oléique à 2 moles d'oxyde d'éthylène vendue sous la dénomination commerciale ETHOMEEN O12 par la société AKZO | 7,0 g |
| - Laurylamino succinamate de diéthylaminopropyle, sel de sodium, à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Monoéthanolamine q.s. | pH 9,8 |
| - Eau déminéralisée q.s.p. | 100 g |

Au moment de l'emploi, on a mélangé cette composition tinctoriale avec une quantité égale d'une composition oxydante constituée par une solution d'eau oxygénée à 20 volumes (6 % en poids) et dont le pH a été ajusté entre 1 et 1,5 par 2,5 g d'acide orthophosphorique pour 100 g d'eau oxygénée.

Le mélange résultant présentait un pH de 6,2 et a été appliqué pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans une nuance bleu cendré.

**EXEMPLES 4 (Invention) et 5 (Comparatif)**

On a préparé les compositions tinctoriales 4 et 5 suivantes :

Composition tinctoriale 4 conforme à l'invention :

| | | |
|---|---|---|
| - 3-fluoro 4-aminophénol | 0,508 | g |
| - 4-hydroxyindole | 0,520 | g |
| - Support de teinture commun défini aux exemples 1 et 2 ci-dessus | (*) | g |
| - Eau déminéralisée q.s.p. | 100 | g |

Composition tinctoriale 5 ne faisant pas partie de l'invention :

| | | |
|---|---|---|
| - Para-aminophénol | 0,5 | g |
| - 4-hydroxyindole | 0,520 | g |
| - Support de teinture commun défini aux exemples 1 et 2 ci-dessus | (*) | g |
| - Eau déminéralisée q.s.p. | 100 | g |

(*) support de teinture commun :

| | | |
|---|---|---|
| - Alcool oléique polyglycérolé à 2 moles de glycérol | 4,0 | g |
| - Alcool oléique polyglycérolé à 4 moles de glycérol, à 78 % de matières actives (M.A.) | 5,69 | g M.A. |
| - Acide oléique | 3,0 | g |
| - Amine oléique à 2 moles d'oxyde d'éthylène vendue sous la dénomination commerciale ETHOMEEN O12 par la société AKZO | 7,0 | g |
| - Laurylamino succinamate de diéthylaminopropyle, sel de sodium, à 55 % de M.A. | 3,0 | g M.A. |
| - Alcool oléique | 5,0 | g |
| - Diéthanolamide d'acide oléique | 12,0 | g |
| - Propylèneglycol | 3,5 | g |

| | |
|---|---|
| - Alcool éthylique | 7,0 g |
| - Dipropylèneglycol | 0,5 g |
| - Monométhyléther de propylèneglycol | 9,0 g |
| - Métabisulfite de sodium en solution aqueuse, à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de $NH_3$ | 10,0 g |

Au moment de l'emploi, on a mélangé chaque composition tinctoriale avec une quantité égale d'une composition oxydante constituée par une solution d'eau oxygénée à 20 volumes (6 % en poids).

Chaque mélange résultant a été appliqué pendant 30 minutes sur des mèches de cheveux gris naturels à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Les mèches de cheveux ainsi teintes ont ensuite été soumises à un test de résistance à la transpiration.

Pour ce faire, les mèches de cheveux ont été immergées dans un cristallisoir recouvert d'un verre de montre et renfermant une solution de sueur synthétique de composition suivante :

| | |
|---|---|
| - NaCl | 10 g |
| - Hydrogénophosphate de potassium | 1 g |
| - Histidine | 0,25 g |
| - Acide lactique q.s. | pH 3,2 |
| - Eau distillée q.s.p. | 100 g |

On a laissé séjourner les mèches de cheveux teints dans cette solution de sueur synthétique pendant 48 heures à 37° C. Les mèches ont ensuite été rincées puis séchées.

La couleur des mèches a été ensuite évaluée à nouveau dans le système MUNSELL au moyen d'un colorimètre CM 2002 MINOLTA de façon à déterminer la dégradation des colorations après ce test de résistance à la transpiration.

Selon la notation MUNSELL, une couleur est définie par l'expression **HV/C** dans laquelle les trois paramètres désignent respectivement la teinte ou Hue **(H)**, l'intensité ou Value **(V)** et la pureté ou Chromaticité **(C)**, la barre oblique de cette expression est simplement une convention et n'indique pas un ratio.

La différence de couleur entre deux mèches est calculée en appliquant la formule de NICKERSON : $\Delta E = 0,4\ Co\Delta H + 6\Delta V + 3\ \Delta C$ , telle que décrite par exemple dans "Couleur, Industrie et Technique"; pages 14-17 ; vol. n° 5; 1978.

Dans cette formule, $\Delta E$ représente la différence de couleur entre deux mèches, $\Delta H$, $\Delta V$ et $\Delta C$ représentent la variation en valeur absolue des paramètres H, V et C et Co représente la pureté de la mèche par rapport à laquelle on désire évaluer la différence de couleur.

Les résultats sont donnés dans le tableau ci-dessous :

| EXEMPLE (COMPOSITION) | Couleur des cheveux avant le test | Couleur des cheveux après le test | Dégradation de la couleur | | | |
|---|---|---|---|---|---|---|
| | | | ΔH | ΔV | ΔC | ΔE |
| **4 (4)** | 0,1 YR 4,4 / 3,3 | 1,2 YR 4,1 / 3,0 | 1,1 | 0,3 | 0,3 | **4,15** |
| **5 (5)** | 9,7 R 4,2 / 3,2 | 0,4 YR 3,3 / 2,3 | 0,7 | 0,9 | 0,9 | **9** |

Ces résultats montrent que la composition de l'exemple 4 conforme à l'invention conduit à une coloration résistant beaucoup mieux à la transpiration que la composition de l'exemple 5 ne faisant pas partie de l'invention et telle que décrite par exemple dans la demande de brevet allemand DE 3 031 709.

**Revendications**

**1.** Composition pour la teinture d'oxydation des fibres kératiniques et en particulier des fibres kératiniques humaines telles que les cheveux, caractérisée par le fait qu'elle comprend, dans un milieu approprié pour la teinture :

- au moins un précurseur de colorant d'oxydation choisi parmi le 3-fluoro 4-aminophénol, le N,N'-bis-(β-hydroxyéthyl) N,N'-bis-(4'-aminophényl) 1,3-diamino 2-propanol et leurs sels d'addition avec un acide ;
- du 4-hydroxyindole, à titre de coupleur.

**2.** Composition selon la revendication 1, caractérisée par le fait que les sels d'addition avec un acide sont choisis parmi les chlorhydrates, les bromhydrates, les sulfates et les tartrates.

**3.** Composition selon la revendication 1 ou 2, caractérisée par le fait que le ou les précurseurs de colorants d'oxydation représentent de 0,0005 à 10 % en poids du poids total de la composition tinctoriale.

**4.** Composition selon la revendication 3, caractérisée par le fait que le ou les précurseurs de colorants d'oxydation représentent de 0,01 à 5 % en poids du poids total de la composition tinctoriale.

**5.** Composition selon l'une quelconque des revendications 1 à 4, caractérisée par le fait que le 4-hydroxyindole représente de 0,0001 à 3,5 % en poids du poids total de la composition tinctoriale.

**6.** Composition selon la revendication 5, caractérisée par le fait que le 4-hydroxyindole représente de 0,005 à 1 % en poids du poids total de la composition tinctoriale.

**7.** Composition selon l'une quelconque des revendications 1 à 6, caractérisée par le fait que ledit milieu approprié pour la teinture est constitué par de l'eau ou par un mélange d'eau et d'au moins un solvant organique choisi parmi les alcanols inférieurs en $C_1$-$C_4$, le glycérol, les glycols et éthers de glycols, les alcools aromatiques, les produits analogues et leurs mélanges.

**8.** Composition selon l'une quelconque des revendications 1 à 7, caractérisée par le fait qu'elle présente un pH compris entre 3 et 12.

**9.** Composition selon l'une quelconque des revendications 1 à 8, caractérisée par le fait qu'elle contient, en outre, d'autres coupleurs différents du 4-hydroxyindole et/ou des colorants directs.

**10.** Procédé de teinture des fibres kératiniques et en particulier des fibres kératiniques humaines telles que les cheveux caractérisé par le fait que l'on applique sur ces fibres une composition tinctoriale telle que définie à l'une quelconque des revendications 1 à 9 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

**11.** Procédé selon la revendication 10, caractérisé par le fait que l'agent oxydant est choisi parmi le peroxyde d'hydrogène, le peroxyde d'urée, les bromates de métaux alcalins, les persels tels que les perborates et persulfates.

12. Dispositif à plusieurs compartiments ou "kit" de teinture à plusieurs compartiments dont un premier compartiment renferme une composition tinctoriale telle que définie à l'une quelconque des revendications 1 à 9 et un second compartiment renferme une composition oxydante.

## Claims

1. Composition for the oxidation dyeing of keratinous fibres, and especially human keratinous fibres such as hair, characterized in that it comprises, in a medium suitable for dyeing:

   - at least one oxidation dye precursor chosen from 3-fluoro-4-aminophenol and N,N'-bis(β-hydroxyethyl)-N,N'-bis(4'-aminophenyl)-1,3-diamino-2-propanol and their addition salts with an acid;
   - 4-hydroxyindole, as coupler.

2. Composition according to Claim 1, characterized in that the addition salts with an acid are chosen from the hydrochlorides, hydrobromides, sulphates and tartrates.

3. Composition according to Claim 1 or 2, characterized in that the oxidation dye precursor or precursors represent(s) from 0.0005 to 10 % by weight of the total weight of the dyeing composition.

4. Composition according to Claim 3, characterized in that the oxidation dye precursor or precursors represent(s) from 0.01 to 5 % by weight of the total weight of the dyeing composition.

5. Composition according to any one of Claims 1 to 4, characterized in that the 4-hydroxyindole represents from 0.0001 to 3.5 % by weight of the total weight of the dyeing composition.

6. Composition according to Claim 5, characterized in that the 4-hydroxyindole represents from 0.005 to 1 % by weight of the total weight of the dyeing composition.

7. Composition according to any one of Claims 1 to 6, characterized in that the said medium suitable for dyeing consists of water or of a mixture of water and at least one organic solvent chosen from $C_1$-$C_4$ lower alkanols, glycerol, glycols and glycol ethers, aromatic alcohols, similar products and mixtures thereof.

8. Composition according to any one of Claims 1 to 7, characterized in that it has a pH of between 3 and 12.

9. Composition according to any one of Claims 1 to 8, characterized in that it contains, in addition, other couplers different from 4-hydroxyindole and/or direct dyes.

10. Process for the dyeing of keratinous fibres, and especially human keratinous fibres such as hair, characterized in that a dyeing composition as defined in any one of Claims 1 to 9 is applied to these fibres, and in that the colour is developed at acid, neutral or alkaline pH using an oxidizing agent which is added to the dyeing composition just at the time of use or which is present in an oxidizing composition applied simultaneously or sequentially in a separate stage.

11. Process according to Claim 10, characterized in that the oxidizing agent is chosen from hydrogen peroxide, urea peroxide, alkali metal bromates and persalts such as perborates and persulphates.

12. Multi-compartment device or multi-compartment dyeing "kit" in which a first compartment contains a dyeing composition as defined in any one of Claim 1 to 9 and a second compartment contains an oxidizing composition.

## Patentansprüche

1. Zusammensetzung zum oxidativen Färben von Keratinfasern und insbesondere von menschlichen Keratinfasern wie dem Haar,
   dadurch gekennzeichnet, daß
   sie in einem zum Färben geeigneten Medium

   - mindestens ein Farbstoffvorprodukt eines Oxidationsfarbstoffes, das unter 3-Fluor-4-aminophenol und N,N'-bis-(β-hydroxyethyl)-N,N'-bis-(4'-aminophenyl)-1,3-diamino-2-propanol sowie den Additionssalzen dieser Verbindungen mit einer Säure ausgewählt ist, und

- 4-Hydroxyindol als Kuppler

enthält.

2. Zusammensetzung nach Anspruch 1, dadurch gekennzeichnet, daß die Additionssalze mit einer Säure unter den Hydrochloriden, Hydrobromiden, Sulfaten und Tartraten ausgewählt sind.

3. Zusammensetzung nach Anspruch 1 oder 2, dadurch gekennzeichnet, daß der oder die Farbstoffvorprodukte von Oxidationsfarbstoffen 0,0005 bis 10 Gew.-% des Gesamtgewichts der Färbemittelzusammensetzung ausmachen.

4. Zusammensetzung nach Anspruch 3, dadurch gekennzeichnet, daß das oder die Farbstoffvorprodukte von Oxidationsfarbstoffen 0,01 bis 5 Gew.-% des Gesamtgewichts der Färbemittelzusammensetzung ausmachen.

5. Zusammensetzung nach einem der Ansprüche 1 bis 4, dadurch gekennzeichnet, daß das 4-Hydroxyindol 0,0001 bis 3,5 Gew.-% des Gesamtgewichts der Färbemittelzusammensetzung ausmacht.

6. Zusammensetzung nach Anspruch 5, dadurch gekennzeichnet, daß das 4-Hydroxyindol 0,005 bis 1 Gew.-% des Gesamtgewichts der Färbemittelzusammensetzung ausmacht.

7. Zusammensetzung nach einem der Ansprüche 1 bis 6, dadurch gekennzeichnet, daß das zum Färben geeignete Medium aus Wasser oder einem Gemisch aus Wasser und mindestens einem organischen Lösungsmittel besteht, das unter den niederen Alkanolen mit 1 bis 4 Kohlenstoffatomen, Glycerin, Glykolen und Glykolethern, aromatischen Alkoholen, analogen Produkten und deren Gemischen ausgewählt ist.

8. Zusammensetzung nach einem der Ansprüche 1 bis 7, dadurch gekennzeichnet, daß sie einen pH-Wert im Bereich von 3 bis 12 aufweist.

9. Zusammensetzung nach einem der Ansprüche 1 bis 8, dadurch gekennzeichnet, daß sie ferner weitere von 4-Hydroxyindol verschiedene Kuppler und/oder Direktfarbstoffe enthält.

10. Verfahren zum Färben von Keratinfasern und insbesondere von menschlichen Keratinfasern, wie dem Haar, dadurch gekennzeichnet, daß auf die Fasern eine Färbemittelzusammensetzung nach einem der Ansprüche 1 bis 9 aufgetragen und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

11. Verfahren nach Anspruch 10, dadurch gekennzeichnet, daß das Oxidationsmittel unter Wasserstoffperoxid, Harnstoffperoxid, Alkalimetallbromaten, Salzen von Persäuren, wie Perboraten und Persulfaten, ausgewählt ist.

12. Vorrichtung mit mehreren Abteilungen oder "Kit" zum Färben mit mehreren Abteilungen, wobei eine erste Abteilung eine Färbemittelzusammensetzung nach einem der Ansprüche 1 bis 9 und eine zweite Abteilung eine oxidierende Zusammensetzung enthält.